# EUROPEAN PATENT APPLICATION

(11) **EP 1 645 981 A2**
(43) Date of publication of application: **12.04.2006**
(21) Application number: 05020265.4
(22) Date of filing: 16.09.2005
(51) Int. Cl.: G06F 19/00

(54) **System for extracting compound differently interacting with bioactive polymer of interest**

(30) Priority: 21.09.2004 JP 2004273359
(71) Applicant: FUJI PHOTO FILM CO., LTD, Kanagawa-ken (JP)
(72) Inventor: Muraishi, Katsuaki c/o Fuji Photo Film Co., Ltd., Ashigarakami-gun Kanagawa-ken (JP); Tsuzuki, Hirohiko, Minamiashigara-shi Kanagawa-ken (JP)
(74) Representative: Klunker . Schmitt-Nilson . Hirsch

(57) **Abstract**

In a compound-extraction system (100) : a storage unit (10) stores first data sets (D1, D2, ... ) each indicating interactions between one of bioactive polymers (T) for reference and predetermined compounds (J) under consideration, in association with the bioactive polymers (T) for reference and the predetermined compounds (J) under consideration; and an extraction unit (20) receives a second data set (E) indicating interactions between a bioactive polymer (U) of interest and the predetermined compounds (J) under consideration, and at least one of the first data sets (D1, D2, ... ) indicating interactions between at least one of the bioactive polymers (T) for reference and the predetermined compounds (J) under consideration, and extracts at least one of the predetermined compounds (J) under consideration which interacts with the bioactive polymer (U) of interest differently than with the predetermined compounds (J) under consideration.

## Description

### BACKGROUND OF THE INVENTION

### Field of the Invention

The present invention relates to a compound-extraction system for extracting, from a plurality of predetermined compounds, a compound which interacts with a polymer of interest differently than with polymers for reference. In addition, the present invention also relates to a compound-extraction program which makes a computer execute a process for extracting, from a plurality of predetermined compounds, a compound which interacts with a polymer of interest differently than with polymers for reference.

### Description of the Related Art

Generally, many compounds which constitute a medicine (medical drug) perform their functions by being bound by a protein in an organism. Therefore, in development of a medicine, it is important to know the strengths of the bindings between proteins and compounds as candidates for the medicine. In particular, it is desirable that the compound constituting a medicine have an affinity for only a protein of interest, and no affinity for other proteins, because an affinity for a protein other than the protein of interest can cause an adverse drug reaction. Thus, in development of a medicine, screening is performed for extracting a compound which has an affinity for only the protein of interest among candidate compounds for the medicine.

Various systems have been proposed for use in the screening of candidate compounds for a medicine. For example, a first known measurement system which has been proposed for the above purpose utilizes a phenomenon in which the attenuated total reflection angle (i.e., a specific reflection angle at which attenuated total reflection occurs) varies with the permittivity in the vicinity of a surface of metal when a light beam is totally reflected at the other surface of the metal so as to generate a surface plasmon wave. The attenuated total reflection is rapid attenuation of the optical intensity of the reflected light beam.

In addition, a second known measurement system which has been proposed for the aforementioned purpose is the leakage-mode measurement system which is disclosed, for example, in "Surface Refracto-sensor using Evanescent Waves: Principles and Instrumentations" by Takayuki Okamoto, Spectrum Researches, Vol. 47, No. 1 ,1998, pp. 21-23 & 26-27. The leakage-mode measurement system also utilizes the attenuated total reflection, and is similar to the first known measurement system. Specifically, the leakage-mode measurement system includes: a dielectric block having a prismatic shape; a cladding layer formed on a surface of the dielectric block; an optical waveguide layer formed on the cladding layer so that the optical waveguide layer can be in contact with a sample solution; a light source which produces a light beam; an optical system which injects the light beam into the dielectric block at various incident angles so that the light beam is totally reflected at the boundary between the dielectric block and the cladding layer, and attenuated total reflection (ATR) due to excitation of a propagation mode in the optical waveguide layer can occur; and a light detection unit which can detect the attenuated total reflection (i.e., the state in which the propagation mode is excited) by measuring the intensity of the light beam totally reflected at the above boundary.

Further, a third known measurement system which utilizes the principle of the surface plasmon and has been proposed for the aforementioned purpose is the BIACORE 2000 system, which is available from Biacore International AB. The use of the BIACORE 2000 system for the aforementioned purpose is disclosed in "Methods of Experiments and Real-time Analysis of Interaction with Biological Material" by K. Nagata and H. Handa, Springer-Verlag Tokyo, 1998. When the BIACORE 2000 system is used, it is possible to obtain results of kinetic analyses of reactions between proteins and compounds (i.e., the rates of binding or dissociating reactions), and extract a desired compound as a candidate for a medicine, from a plurality of compounds, on the basis of the obtained results.

In an example of extraction of a compound as a candidate for a medicine by use of the BIACORE 2000 system is disclosed in "Simultaneous Analysis of Drug Efficacy and Early Absorption, Distribution, Metabolism and Excretion Characterization Using Surface Plasmon Resonance Sensor," by Jinichi Inagawa and Kaori Morimoto, Bunseki Kagaku, Vol. 51, No. 6, pp. 461-468, The Japan Society for Analytical Chemistry, 2002. In this example, two types of proteins are immobilized on a sensor chip, and compounds as candidates for a medicine are narrowed down by making measurements on interactions and the like between each type of proteins and thirty-four compounds (e.g., measurements of the rates of binding or dissociating reactions or the amounts of chemical bindings).

However, in the screening for extracting a compound as a candidate for a medicine from a great number of compounds, the number of proteins actually referred to is approximately several hundreds to several thousands, and the number of compounds under consideration is approximately several millions. Nevertheless, in the aforementioned conventional systems, interactions between a protein and a plurality of compounds under consideration, which are stored in advance, are compared with interactions between a bioactive polymer of interest and the plurality of compounds under consideration. Each of the above conventional systems is arranged so that each system can store information on interactions between only one protein and a plurality of compounds. Therefore, when one of the above conventional systems is used and a great number of proteins are referred to, it takes much time to extract a compound as a candidate for a medicine. That is, when the conventional systems are used, the working efficiency is low.

### SUMMARY OF THE INVENTION

The present invention has been developed in view of the above circumstances.

The first object of the present invention is to provide a compound-extraction system which can efficiently extract a desired compound from a plurality of predetermined compounds under consideration.

The second object of the present invention is to provide a compound-extraction program which makes a computer execute a process for efficiently extracting a desired compound from a plurality of predetermined compounds.

The third object of the present invention is to provide a computer-readable medium storing a compound-extraction program which makes a computer execute a process for efficiently extracting a desired compound from a plurality of predetermined compounds.

In order to accomplish the aforementioned first object, the first aspect of the present invention is also provided. According to the first aspect of the present invention, there is provided a compound-extraction system comprising: a storage unit which stores first data sets each indicating interactions between one of a plurality of bioactive polymers for reference and a plurality of predetermined compounds under consideration, in association with the plurality of bioactive polymers for reference and the plurality of predetermined compounds under consideration; an extraction unit which receives a second data set indicating interactions between a bioactive polymer of interest and the plurality of predetermined compounds under consideration, and at least one of the first data sets indicating interactions between at least one of the plurality of bioactive polymers for reference and the plurality of predetermined compounds under consideration, and extracts, on the basis of the second data set and the at least one of the first data sets, at least one of the plurality of predetermined compounds under consideration which interacts with the bioactive polymer of interest differently than with the plurality of bioactive polymers for reference; and an output unit which outputs information indicating the at least one of the plurality of predetermined compounds under consideration which is extracted by the extraction unit.

In addition, in order to accomplish the aforementioned second object, the second aspect of the present invention is provided. According to the second aspect of the present invention, there is provided a compound-extraction program which makes a computer execute a process comprising the steps of: (a) storing, in a storage unit, first data sets each indicating interactions between one of a plurality of bioactive polymers for reference and a plurality of predetermined compounds under consideration, in association with the plurality of bioactive polymers for reference and the plurality of predetermined compounds under consideration; (b) inputting, into an extraction processing unit, a second data set indicating interactions between a bioactive polymer of interest and the plurality of predetermined compounds under consideration; (c) inputting, into the extraction processing unit, at least one of the first data sets indicating interactions between at least one of the plurality of bioactive polymers for reference and the plurality of predetermined compounds under consideration; (d) extracting, on the basis of the second data set and the at least one of the first data sets by the extraction processing unit, at least one of the plurality of predetermined compounds under consideration which interacts with the bioactive polymer of interest differently than with the plurality of bioactive polymers for reference; and (e) outputting information indicating the at least one of the plurality of predetermined compounds under consideration which is extracted by the extraction unit.

Further, in order to accomplish the aforementioned third object, the third aspect of the present invention is provided. According to the third aspect of the present invention, there is provided a computer-readable medium storing the compound-extraction program according to the second aspect of the present invention.

When the compound-extraction system and the compound-extraction program according to the first and second aspects of the present invention are used, the comparison can be made between a bioactive polymer of interest and a plurality of bioactive polymers for reference, although the comparison can be made between a bioactive polymer of interest and only one bioactive polymer for reference in the conventional systems. Therefore, it is possible to increase the efficiency in extraction of at least one desired compound (i.e., at least one of the plurality of predetermined compounds under consideration which interacts with the bioactive polymer of interest differently than with the plurality of bioactive polymers for reference).

The bioactive polymers handled in the present invention include the following materials:
(i) The natural polymers Nos. 11001 to 11025 indicated in Japanese Industrial Standards (JIS) K3611 (in Japanese)
(ii) The sugar, amino acids, nucleotides, lipids, hemes, quinones, proteins, RNAs, DNAs, phospholipids, and polysaccharides indicated in Chapter II, Section 1, "Survey of Biological Materials" in "Encyclopedia of Biological Data (in Japanese)," edited by K. Ishihara et al. and published by Asakura Shoten in 2002
(iii) The proteins, amino acids, nucleic acids, lipids, carbohydrates, and enzymes indicated in Chapter 2, "Biological Materials and Metabolism" in "Encyclopedia of Bioscience (in Japanese)," edited by J. Ohta and published by Asakura Shoten in 2001

Each of the interactions handled in the present invention is indicated by one or a combination of two or more of the rate of a binding reaction, the amount of binding, and the rate of a dissociation reaction, of a physical, chemical, or biochemical reaction.

Each of the compounds handled in the present invention is a molecule constituted by two or more (chemical) elements. Although the molecular weights of the compounds handled in the present invention are mainly in the range from 50 to a hundred million, the molecular weights of the compounds handled in the present invention are not limited to the range.

The second data set indicating interactions between a bioactive polymer of interest and the plurality of predetermined compounds under consideration may also be stored in the storage unit.

The first and second data sets may be obtained by using a measurement system which utilizes the attenuated total reflection. Such a measurement system utilizes the aforementioned phenomenon in which the attenuated total reflection angle varies with the permittivity in the vicinity of a surface of metal when a light beam is totally reflected at the other surface of the metal so as to generate a surface plasmon wave.

Each of the first and second data sets includes at least one value of at least one of a binding-rate constant Ka, a dissociation-rate constant Kd, a dissociation constant KD, and a maximum binding Rmax. The binding-rate constant Ka indicates the rate of a chemical binding reaction, the dissociation-rate constant Kd indicates the rate of a chemical dissociation reaction, the dissociation constant KD is the dissociation-rate constant Kd divided by the binding-rate constant Ka, and the maximum binding Rmax is the saturated amount of chemical binding between a compound and a bioactive polymer.

### BRIEF DESCRIPTION OF THE DRAWINGS

FIG. 1 is a diagram schematically illustrating a construction of a compound-extraction system according to an embodiment of the present invention.
FIG. 2 is a diagram illustrating an example of the format of a data set stored in the database.
FIGS. 3A, 3B, and 3C are diagrams each illustrating examples of data sets indicating interactions of compounds under consideration, with a bioactive polymer of interest and a plurality of bioactive polymers for reference.
FIG. 4A is a diagram illustrating an example of display of a result of extraction of a compound in accordance with an extraction condition 1.
FIG. 4B is a diagram illustrating an example of display of a result of extraction of a compound in accordance with an extraction condition 2.
FIGS. 5A and 5B are diagrams each illustrating other examples of data sets indicating interactions of compounds under consideration, with a bioactive polymer of interest and a plurality of bioactive polymers for reference.
FIG. 6 is a diagram illustrating an example of display of a result of extraction of a compound in accordance with an extraction condition 3.

### DESCRIPTION OF THE PREFERRED EMBODIMENTS

An embodiment of the present invention is explained in detail below with reference to the drawings.

### Compound-extraction System

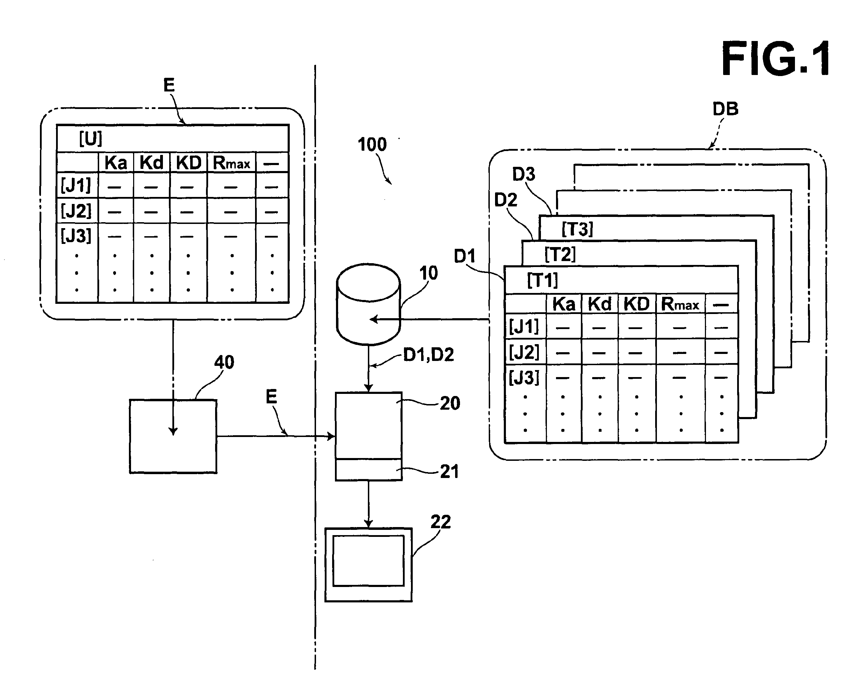

FIG. 1 is a diagram schematically illustrating a construction of a compound-extraction system according to an embodiment of the present invention.

As illustrated in FIG. 1, the compound-extraction system 100 according to the embodiment of the present invention comprises a database unit 10, a compound-extraction unit 20, an output unit 21, and a display unit 22.

The database unit 10 stores first data sets D1, D2, ... each indicating interactions between one of a plurality of proteins T1, T2, ... (as bioactive polymers for reference) and a plurality of predetermined compounds J1, J2, ... under consideration, in association with the plurality of proteins T1, T2, ... and the plurality of predetermined compounds J1, J2, .... Hereinafter, the plurality of proteins T1, T2, ... for reference are collectively referred to as the proteins T, the plurality of predetermined compounds J1, J2, ... under consideration are collectively referred to as the compounds J, and the first data sets D1, D2, ... constitute a database DB.

The compound-extraction unit 20 receives a second data set E indicating interactions between a protein U as a bioactive polymer of interest and the compounds J under consideration, and at least one of the first data sets (two data sets D1 and D2, in this example) indicating interactions between at least one of the proteins T for reference (two proteins T1 and T2, in this example) and the compounds J under consideration, and extracts, on the basis of the second data set E and the at least one of the first data sets D1, D2, ... (the two data sets D1 and D2), at least one of the compounds J under consideration which interacts with the protein U of interest differently than with the at least one of the proteins T for reference (the two proteins T1 and T2).

The output unit 21 outputs a result of the extraction by the compound-extraction unit 20, and the display unit 22 displays the result outputted by the output unit 21.

Alternatively, the compound-extraction unit 20 may be arranged to receive the second data set E and only one of the first data sets (e.g., the data set D1) indicating interactions between only one of the proteins T for reference (e.g., the protein T1) and the compounds J under consideration, and extract, on the basis of the second data set E and the one of the first data sets (e.g., the data set D1), at least one of the compounds J under consideration which interacts with the protein U of interest differently than with the protein T1 for reference.

Further, the protein U of interest may be selected from among the proteins T for reference, so that the second data set E may be one of the first data sets D1, D2, ....

The second data set E in this example is obtained by measurement made by using a surface-plasmon-resonance measurement system 40, which utilizes the attenuated total reflection. In addition, the first data sets D1, D2, ... can also be obtained by using the surface-plasmon-resonance measurement system 40.

### Data Format

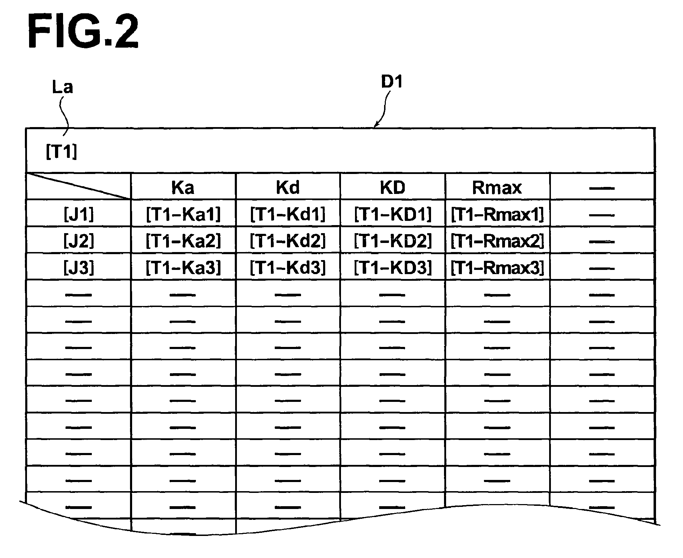

The first data sets D1, D2, ... (for the proteins T1 and T2) and the second data set E (for the protein U) are arranged in similar formats. FIG. 2 is a diagram illustrating an example of the format of a data set stored in the database. In the example of FIG. 2, the data set D1 for the protein T1 is indicated in a tabular form. The table for the data set D1 is labeled "T1" as indicated in a protein-name area La in FIG. 2, and has fields for respective measures of interactions and rows (records) for the respective compounds J under consideration. In this example, the measures of interactions include the binding-rate constant Ka, the dissociation-rate constant Kd, the dissociation constant KD, and the maximum binding Rmax. Thus, the values T1-Kal, T1-Ka2, ... of the binding-rate constant Ka which respectively indicate interactions between the protein T1 and the compounds J, the values T1-Kdl, T1-Kd2, ... of the dissociation-rate constant Kd which respectively indicate interactions between the protein T1 and the compounds J, the values T1-KD1, T1-KD2, ... of the dissociation constant KD which respectively indicate interactions between the protein T1 and the compounds J, and the values T1-Rmax1, T1-Rmax2, ... of the maximum binding Rmax which respectively indicate interactions between the protein T1 and the compounds J are indicated in the corresponding cells in the table.

### First Exemplary Operation

Hereinbelow, a first exemplary operation of extracting at least one compound on the basis of the second data set E and two or more of the first data sets D1, D2, ... is explained.

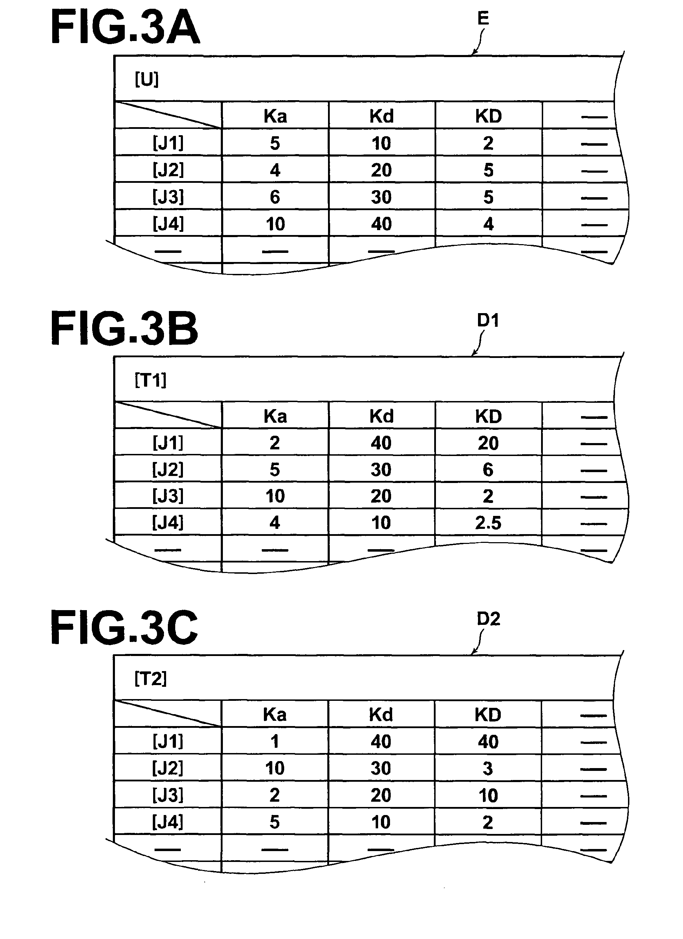

FIG. 3A is a diagram illustrating an example of the data set E which indicates interactions between the protein U of interest and the compounds J under consideration, FIG. 3B is a diagram illustrating an example of the data set D1 which indicates interactions between the protein T1 for reference and the compounds J under consideration, and FIG. 3C is a diagram illustrating an example of the data set D2 which indicates interactions between the protein T2 for reference and the compounds J under consideration. In FIGS. 3A, 3B, and 3C, the values of the binding-rate constant Ka, the dissociation-rate constant Kd, and the dissociation constant KD indicated in the respective fields are chosen for clarification of the explanations, and are not the values obtained by actual measurement. Since the other measures of interactions such as the maximum binding Rmax are not used in the following explanations on the first exemplary operation, the values of only the binding-rate constant Ka, the dissociation-rate constant Kd, and the dissociation constant KD are indicated in FIGS. 3A, 3B, and 3C. In addition, since only the compounds J1, J2, J3, and J4 are considered in the following explanations, only the compounds J1, J2, J3, and J4 are indicated as the compounds J under consideration.

First, the data sets D1 and D2 are inputted from the database unit 10 into the compound-extraction unit 20, and the data set E is also inputted from the surface-plasmon-resonance measurement system 40 into the compound-extraction unit 20. Then, the compound-extraction unit 20 extracts at least one of the compounds J under consideration which interacts with the protein U of interest differently than with the proteins T1 and T2 for reference. That is, the compound-extraction unit 20 extracts at least one of the compounds J which exhibits substantially different values of the binding-rate constant Ka, the dissociation-rate constant Kd, and the dissociation constant KD in the interaction with the protein U than in the interactions with the proteins T1 and T2.

For example, the above extraction can be performed in accordance with one of the following extraction conditions 1 and 2.

The extraction condition 1 is that the binding-rate constant Ka or the dissociation constant KD be greater in the interaction with the protein U of interest than in the interactions with the proteins T1 and T2 for reference, and the dissociation-rate constant Kd be smaller in the interaction with the protein U of interest than in the interactions with the proteins T1 and T2 for reference.

The extraction condition 2 is that the binding-rate constant Ka or the dissociation constant KD be greater in the interaction with the protein U of interest than in the interactions with the proteins T1 and T2 for reference, or the dissociation-rate constant Kd be smaller in the interaction with the protein U of interest than in the interactions with the proteins T1 and T2 for reference.

In the case where the data sets E, D1, and D2 have the values as illustrated in FIGS. 3A, 3B, and 3C, only the proteins J1 and J4 satisfy the first part of the extraction condition 1 or 2 that the binding-rate constant Ka or the dissociation constant KD be greater in the interaction with the protein U of interest than in the interactions with the proteins T1 and T2 for reference, and only the proteins J1 and J2 satisfy the second part of the extraction condition 1 or 2, i.e., have a smaller value of the dissociation-rate constant Kd in the interaction with the protein U of interest than in the interactions with the proteins T1 and T2 for reference.

Therefore, only the compound J1 satisfies the extraction condition 1. Thus, in the case where the extraction is performed in accordance with the extraction condition 1, the compound-extraction unit 20 extracts the compound J1 as one of the compounds J1, J2, ... which exhibits substantially different values of the binding-rate constant Ka, the dissociation-rate constant Kd, and the dissociation constant KD in the interaction with the protein U than in the interactions with the proteins T1 and T2.

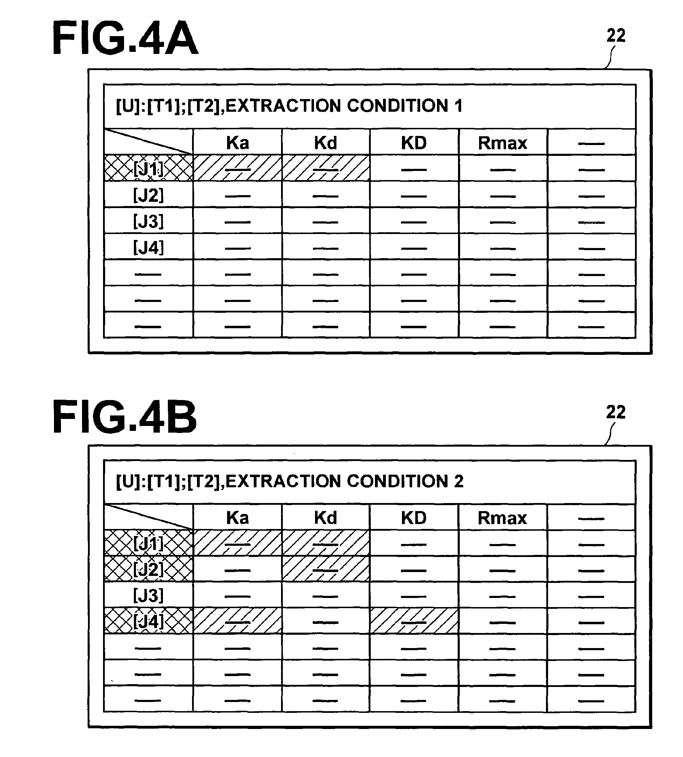

The output unit 21 outputs the above result of the extraction in accordance with the extraction condition 1, and the display unit 22 displays the result as illustrated in FIG. 4A, which shows an example of display of the result of the extraction in accordance with the extraction condition 1. In the table displayed on the display unit 22, the area in which the name of the compound J1 is indicated is colored differently from the areas in which the names of the other compounds J2, J3, and J4 are indicated, and the cells in the row of the compound J1 corresponding to the measures of interaction which meet the extraction condition 1 (i.e., the binding-rate constant Ka and the dissociation-rate constant Kd) are also colored differently from the other cells.

On the other hand, the compounds J1, J2, and J4 satisfy the extraction condition 2. Thus, in the case where the extraction is performed in accordance with the extraction condition 2, the compound-extraction unit 20 extracts the compounds J1, J2, and J4, and the result of the extraction in accordance with the extraction condition 2 is outputted by the output unit 21, and displayed by the display unit 22 as illustrated in FIG. 4B, which shows an example of display of the result of the extraction in accordance with the extraction condition 2. In the table displayed on the display unit 22, the areas in which the names of the compounds J1, J2, and J4 are indicated are colored differently from the area in which the name of the other compound J1 is indicated, and one or more cells in each of the rows of the compounds J1, J2, and J4 corresponding to one or more measures of interaction which meet the extraction condition 2 are also colored differently from the other cells. In this case, the cells in the row of the compound J1 corresponding to the binding-rate constant Ka and the dissociation-rate constant Kd, the cell in the row of the compound J2 corresponding to the dissociation-rate constant Kd, and the cells in the row of the compound J4 corresponding to the binding-rate constant Ka and the dissociation constant KD are differently colored.

As explained above, in the case where two or more data sets indicating interactions between two or more bioactive polymers for reference and a plurality of predetermined compounds under consideration are inputted onto the compound-extraction unit, the compound-extraction unit can extract at least one of the plurality of predetermined compounds under consideration which interacts with a bioactive polymer of interest differently than with the two or more bioactive polymers for reference. In such a case, it is possible to extract, as the at least one of the plurality of predetermined compounds, at least one compound which interacts with the bioactive polymer of interest in a manner different from a manner in which it interests with each of the two or more bioactive polymers for reference.

Further, the interaction of each compound and each bioactive polymer may be indicated by only one or a combination of the binding-rate constant Ka, the dissociation-rate constant Kd, the dissociation constant KD, and the maximum binding Rmax.

### Second Exemplary Operation

Hereinbelow, a second exemplary operation of extracting at least one compound on the basis of the second data set E and only one of the first data sets D1, D2, ... is explained.

In this case, the compound-extraction unit 20 receives the second data set E indicating interactions between a protein U as a bioactive polymer of interest and the compounds J under consideration, and for example, the data set D1 (out of the first data sets D1, D2, . . .) indicating interactions between the protein T1 for reference and the compounds J under consideration, and extracts, on the basis of the second data set E and the data set D1, at least one of the compounds J under consideration which interacts with the protein U of interest differently than with the protein T1 for reference, where the second data set E is obtained by measurement using the surface-plasmon-resonance measurement system 40.

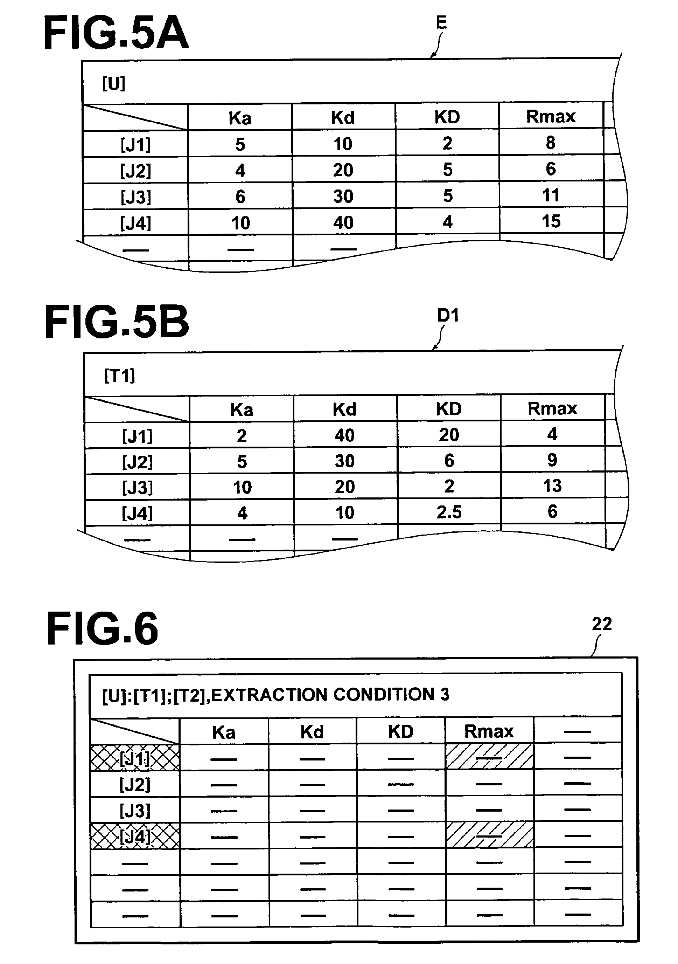

FIG. 5A is a diagram illustrating an example of the data set E which indicates interactions between the protein U of interest and the compounds J under consideration, and FIG. 5B is a diagram illustrating an example of the data set D1 which indicates interactions between the protein T1 for reference and the compounds J under consideration. In FIGS. 5A and 5B, the values of the binding-rate constant Ka, the dissociation-rate constant Kd, the dissociation constant KD, and the maximum binding Rmax indicated in the respective fields are chosen for clarification of the explanations on the second exemplary operation, and are not the values obtained by actual measurement.

For example, the above extraction can be performed in accordance with the extraction condition 3 that the maximum binding Rmax be greater in an interaction with the protein U of interest than in an interaction with the protein T1 for reference.

In the case where the data sets E and D1 have the values as illustrated in FIGS. 5A and 5B, only the proteins J1 and J4 satisfy the extraction condition 3. Therefore, in the case where the extraction is performed in accordance with the extraction condition 3, the compound-extraction unit 20 extracts the compounds J1 and J4 as compounds which satisfy the extraction condition 3.

FIG. 6 is a diagram illustrating an example of display of a result of extraction of a compound.

The output unit 21 outputs the above result of the extraction in accordance with the extraction condition 3, and the display unit 22 displays the result as illustrated in FIG. 6, which shows an example of display of the result of the extraction in accordance with the extraction condition 3. In this case, in the table displayed on the display unit 22, the areas in which the names of the compounds J1 and J4 are indicated are colored differently from the areas in which the names of the other compounds J2 and J3 are indicated, and the cells in the rows of the compounds J1 and J4 corresponding to the measure of interaction which meets the extraction condition 3 (i. e. , the maximum binding Rmax) are also colored differently from the other cells.

As explained above, according to the present invention, it is possible to efficiently extract, from a plurality of predetermined compounds under consideration, at least one compound which interacts with a bioactive polymer of interest differently than with at least one bioactive polymer for reference.

### Variations and Other Matters

(i) Each of the bioactive polymers may include one or a combination of proteins, DNAs, RNAs, and polysaccharides.
(ii) Each of the interactions may be indicated by one or a combination of two or more of the binding-rate constant Ka, the dissociation-rate constant Kd, the dissociation constant KD, and the maximum binding Rmax.
(iii) The first data sets stored in the storage unit in the compound-extraction system according to the present invention may be obtained by luminescence measurement, fluorescence measurement, absorption measurement, ultraviolet absorption measurement, time-resolved fluorescence measurement, fluorescence polarization measurement, or the like, as well as the measurement using the leakage-mode measurement system which utilizes the attenuated total reflection.
(iv) The second data set indicating interactions between a bioactive polymer of interest and the plurality of predetermined compounds under consideration may also be obtained by luminescence measurement, fluorescence measurement, absorption measurement, ultraviolet absorption measurement, time-resolved fluorescence measurement, fluorescence polarization measurement, or the like, as well as the measurement using leakage-mode measurement system which utilizes the attenuated total reflection.
(v) The compound-extraction system according to the present invention can be realized by a computer. In this case, a program (i.e., a compound-extraction program) describing details of the processing which realizes the functions of the compound-extraction system is provided. When the computer executes the program, the functions of the compound-extraction system can be realized on the computer.
   The program describing the details of the processing can be stored in a computer-readable medium (i. e., a recording medium which can be read by the computer). The computer-readable medium is not limited to any specific type of storage devices and includes any kind of devices, including but not limited to CDs, floppy disks, RAMs, ROMs, hard disks, magnetic tapes, and internet downloads, in which computer instructions can be stored and/or transmitted. Transmission of computer codes through a network or through wireless transmission means is also within the scope of this invention. Additionally, computer codes/instructions include, but are not limited to, source, object, and executable codes and can be written in any language including higher level languages, assembly languages, and machine languages.
(vi) All of the contents of the Japanese patent application No. 2004-273359 are incorporated into this specification by reference.

## Claims

1. A compound-extraction system for extracting a desired compound from a plurality of compounds under consideration, comprising:
a storage unit (10) which stores first data sets (D1, D2, . . .) each indicating interactions between one of a plurality of bioactive polymers (T1, T2, . . .) for reference and a plurality of predetermined compounds (J1, J2, . . .) under consideration, in association with the plurality of bioactive polymers (T1, T2, . . .) for reference and the plurality of predetermined compounds (J1, J2, . . .) under consideration;
an extraction unit (20) which receives a second data set (E) indicating interactions between a bioactive polymer (U) of interest and said plurality of predetermined compounds (J1, J2, . . .) under consideration, and at least one of the first data sets (D1, D2, . . .) indicating interactions between at least one of said plurality of bioactive polymers (T1, T2, . . .) for reference and said plurality of predetermined compounds (J1, J2, . . .) under consideration, and extracts, on the basis of the second data set (E) and the at least one of the first data sets (D1, D2, . . .), at least one of said plurality of predetermined compounds (J1, J2, . . .) under consideration which interacts with said bioactive polymer (U) of interest differently than with said plurality of bioactive polymers (T1, T2, . . .) for reference; and
an output unit (21) which outputs information indicating said at least one of the plurality of predetermined compounds (J1, J2, . . .) under consideration which is extracted by said extraction unit (20).

2. A compound-extraction system according to claim 1, wherein said first data sets (D1, D2, . . .) and said second data set (E) are obtained by using a measurement system (40) which utilizes attenuated total reflection.

3. A compound-extraction system according to either of claims 1 and 2, wherein each of said first data sets (D1, D2, . . .) and said second data set (E) includes at least one value of at least one of a binding-rate constant (Ka), a dissociation-rate constant (Kd), a dissociation constant (KD), and a maximum binding (Rmax).

4. A compound-extraction program which makes a computer execute a process for extracting a desired compound from a plurality of compounds under consideration, said process comprising the steps of:
(a) storing, in a storage unit (10), first data sets (D1, D2, . . .) each indicating interactions between one of a plurality of bioactive polymers (T1, T2, . . .) for reference and a plurality of predetermined compounds (J1, J2, . . .) under consideration, in association with the plurality of bioactive polymers (T1, T2, . . .) for reference and the plurality of predetermined compounds (J1, J2, . . .) under consideration;
(b) inputting, into an extraction processing unit, a second data set (E) indicating interactions between a bioactive polymer (U) of interest and said plurality of predetermined compounds (J1, J2, . . .) under consideration;
(c) inputting, into said extraction processing unit, at least one of the first data sets (D1, D2, . . .) indicating interactions between at least one of said plurality of bioactive polymers (T1, T2, . . .) for reference and said plurality of predetermined compounds (J1, J2, . . .) under consideration;
(d) extracting, on the basis of the second data set (E) and said at least one of the first data sets (D1, D2, . . .) by said extraction processing unit, at least one of said plurality of predetermined compounds (J1, J2, . . .) under consideration which interacts with said bioactive polymer (U) of interest differently than with said plurality of bioactive polymers (T1, T2, . . .) for reference; and
(e) outputting information indicating said at least one of the plurality of predetermined compounds (J1, J2, . . .) under consideration which is extracted by said extraction unit (20) .

5. A compound-extraction program according to claim 4, wherein said first data sets (D1, D2, . . .) and said second data set (E) are obtained by using a measurement system (40) which utilizes attenuated total reflection.

6. A compound-extraction program according to either of claims 4 and 5, wherein each of said first data sets (D1, D2, . . .) and said second data set (E) includes at least one value of at least one of a binding-rate constant (Ka), a dissociation-rate constant (Kd), a dissociation constant (KD), and a maximum binding (Rmax).

7. A computer-readable medium storing a compound-extraction program which makes a computer execute a process for extracting a desired compound from a plurality of compounds under consideration, said process comprising the steps of:
(a) storing, in a storage unit (10), first data sets (D1, D2, . . .) each indicating interactions between one of a plurality of bioactive polymers (T1, T2, . . .) for reference and a plurality of predetermined compounds (J1, J2, . . .) under consideration, in association with the plurality of bioactive polymers (T1, T2, . . .) for reference and the plurality of predetermined compounds (J1, J2, . . .) under consideration;
(b) inputting, into an extraction processing unit, a second data set (E) indicating interactions between a bioactive polymer (U) of interest and said plurality of predetermined compounds (J1, J2, . . .) under consideration;
(c) inputting, into said extraction processing unit, at least one of the first data sets (D1, D2, . . .) indicating interactions between at least one of said plurality of bioactive polymers (T1, T2, . . .) for reference and said plurality of predetermined compounds (J1, J2, . . .) under consideration;
(d) extracting, on the basis of the second data set (E) and said at least one of the first data sets (D1, D2, . . .) by said extraction processing unit, at least one of said plurality of predetermined compounds (J1, J2, . . .) under consideration which interacts with said bioactive polymer (U) of interest differently than with said plurality of bioactive polymers (T1, T2, . . .) for reference; and
(e) outputting information indicating said at least one of the plurality of predetermined compounds (J1, J2, . . .) under consideration which is extracted by said extraction unit (20) .

8. A computer-readable medium according to claim 7, wherein said first data sets (D1, D2, . . .) and said second data set (E) are obtained by using a measurement system (40) which utilizes attenuated total reflection.

9. A computer-readable medium according to either of claims 7 and 8, wherein each of said first data sets (D1, D2, . . .) and said second data set (E) includes at least one value of at least one of a binding-rate constant (Ka), a dissociation-rate constant (Kd), a dissociation constant (KD), and a maximum binding (Rmax).
